# EUROPEAN PATENT SPECIFICATION

(11) **EP 0 752 884 B1**
(45) Date of publication and mention of the grant of the patent: **28.07.2004**
(21) Application number: 95916293.4
(22) Date of filing: 11.04.1995
(51) Int. Cl.: A61K 38/21, A61P 37/00

(54) **METHOD OF TREATING AUTO-IMMUNE DISEASES USING TYPE ONE INTERFERONS**
VERFAHREN ZUR BEHANDLUNG VON AUTOIMMUNERKRANKUNGEN MITTELS TYP-1-INTERFERONEN
TRAITEMENT DE MALADIES AUTO-IMMUNES A L'AIDE D'INTERFERONS DE TYPE 1

(30) Priority: 12.04.1994 US 226631; 24.03.1995 US 408271
(43) Date of publication of application: 15.01.1997
(73) Proprietor: RESEARCH DEVELOPMENT FOUNDATION, Carson City, Nevada 89703 (US)
(72) Inventor: BROD, Staley Armstrong, Houston, TX 77006-6033 (US)
(74) Representative: Knowles, James Atherton
(86) International application number: PCT/US1995/004420
(87) International publication number: WO 1995/027502

(56) References cited:
- US-A- 5 019 382
- "INTERFERON BETA-1B IS EFFECTIVE IN RELAPSING-REMITTING MULTIPLE SCLEROSIS CLINICAL RESULTS OF A MULTICENTER, RANDOMIZED, DOUBLE-BLIND, PLACEBO-CONTROLLED TRIAL" NEUROLOGY, vol. 43, no. 4, April 1993, pages 655-661, XP000614982
- GOODKIN D.E.: "Experimental therapies for multiple sclerosis: current status" CLEVELAND CLIN.J.MED., vol. 59, no. 1, 1992, pages 63-74, XP002092153 Cleveland
- CHEMICAL ABSTRACTS, Volume 108, Number 1, issued 04 January 1988, YASUNORI SHIBUTANI et al., "Toxicity Studies of Human Fibroblast Interferon beta (I). Acute and Subacute Toxicity Studies in Mice and Rats", abstract no. 4454; & IYAKUHIN KENKYU, Volume 18, No. 4, issued 1987, pages 571-82.
- IMMUNOLOGY TODAY, Volume 8, No. 3, issued 1987, ALLAN MCL. MOWAT, "The Regulation of Immune Responses to Dietary Protein Antigens", pages 93-98.
- JOUR. OF IMMUNOL., Volume 140, No. 2, issued 15 January 1988, PAUL J. HIGGINS et al., "Suppression of Experimental Autoimmune Encephalomyelitis by Oral Administration of Myelin Basic Protein and its Fragments", pages 440-445.
- CLIN. EXP. IMMUNOL., Volume 64, issued 1985, H.S.G. THOMPSON et al., "Gastric Administration of Type II Collagen Delays the Onset and Severity of Collagen-Induced Arthritis in Rats", pages 581-586.

## Description

### BACKGROUND OF THE INVENTION

### Field of the Invention

The present invention relates generally to the fields of neurology, immunology and protein chemistry. More specifically, the present invention relates to a novel methods of treating auto-immune diseases using type one interferons.

### Description of the Related Art

Acute experimental autoimmune encephalomyelitis [EAE] is a T cell mediated inflammatory autoimmune process of the central nervous system which resembles the human disease multiple sclerosis. CR-EAE, a chronic inflammatory autoimmune process of the central nervous system that resembles human multiple sclerosis [MS] both in its pathological and clinical expression, provides a model to assess interventions to alter the course of a human autoimmune disease. Myelin basic protein [MBP] is an important neuroantigen in the pathogenesis of this disease. CR-EAE in SJL/J mice can be adoptively transferred following the intravenous injection of an MBP peptide 89-100 specific T cell line. Coculture of MBP peptide 91-103 specific T cells with chemically crosslinked peptide renders the T cells tolerant to MBP in CR-EAE in the SJL/J mouse.

Parenteral, e.g., IV administration of natural rat interferon [10⁵ units] partially suppresses acute EAE in male Lewis rats and inhibits passive hyperacute localized EAE when administered on the same day of immunogen inoculation. Other parenterally administered cytokines such as TGF-β can decrease clinical disease and inflammation in brain and spinal cord in EAE. Parenterally administered natural human IFN-α can decrease T cell function and T cell dependent antibody production in humans. Orally administered natural human IFN-α can prevent experimental development of viral and parasitic infections in animals. Acute EAE provides a model to demonstrate the ability of orally administered immunoactive substances to influence the course of an autoimmune disease.

Human IFN-α is an immunoactive protein that can be orally administered at low doses in the treatment of viral disease in animals. Human IFN-β, another type 1 interferon, augments suppressor cell function in vitro in progressive MS and rat IFN-β decreases the severity of symptoms in rat EAE. In view of the immunoregulatory and anti-viral properties of the type 1 interferons, its response and production has been assessed in autoimmune diseases. Inactive rheumatoid arthritis is marked by augmented [2'-5'] oligoadenylate synthetase [OAS], a readily assayed measurement of type 1 interferon activity, in peripheral blood leucocytes secondary to an IFN-α/β stimulus; active rheumatoid arthritis exhibited a significantly reduced IFN-α/β production compared to normal donors. Other autoimmune diseases, such as psoriasis and atopic dermatitis, also showed decreased interferon production. Peripheral blood lymphocytes in patients with MS show a similar ineffective production of type 1 interferons in response to viral or mitogenic stimulus which parallels the severity of the disease. Natural killer [NK] cell activity is deficient in MS, which is correlated with disease severity, and can be normalized with IFN-α treatment.

The reported abnormalities of production or response to type 1 interferon in autoimmune diseases have prompted several small pilot studies using type 1 interferons as therapeutic agents in MS. These studies used systemic administered type 1 interferon in doses of 1 million units or more daily without significant clinical improvement. A study of parenterally administered IFN-β in relapsing-remitting MS suggests that 1.6-8 million units of IFN-β s.c., three times per week, can decrease relapses by 40-50% and decrease brain inflammation as assessed by serial MRI.

The prior art is deficient in the lack of effective means of treating auto-immune diseases by oral administration of cytokines, such as type one interferons. The present invention fulfills this longstanding need and desire in the art.

### Summary of the Invention

It is an object of the present invention to show that the oral administration of a type one interferon inhibits proliferation to sensitised antigens, suppresses the clinical expression of relapses, and decreases inflammation via alteration of cytokines in murine CR-EAE.

It is another object of the present invention to illustrate the effect of type one interferons orally administered before and after immunization with GP-MBP in acute EAE.

It is another object of the present invention to suppress the clinical expression of attacks of autoimmune disease, decrease pathological sequelae and inhibit inflammatory cytokines in such diseases by the oral administration of biological response modifiers such as type one interferons.

It is also an object of the present invention to show the effect of oral rat IFN-α/β on clinical outcome, histology and IFN-γ secretion in experimental allergic neuritis, the *in vivo* model of acute inflammatory demyelinating radiculoneuropathy or Guillain-Barré syndrome.

In one embodiment of the present invention, there is provided the use of a type one interferon in the manufacture of a medicament for use in the treatment of an auto-immune disease by oral administration, characterised in that said interferon in a dosage of from 50IU/kg to 25,000IU/kg and in that said interferon is ingested after administration.

In another embodiment of the present invention, there is provided the use of a type one interferon in the manufacture of a medicament for use in decreasing the severity or frequency of a relapse of multiple sclerosis in a human by oral administration, characterised in that said interferon is ingested after administration.

In yet another embodiment of the present invention, there is provided the use of a type one interferon in the manufacture of a medicament for use in reducing inflammation associated with an auto-immune disease by oral administration, characterised in that said interferon is for ingestion after administration.

In still yet another embodiment of the present invention, there is provided the use of a type one interferon in the manufacture of a medicament for use in inhibiting the onset of an auto-immune disease by oral administration, characterised in that said interferon is ingested after administration and in that said interferon is in a dosage of from 50 IU/kg to 25,000 IU/kg. description of the presently preferred embodiments of the invention given for the purpose of disclosure.

### BRIEF DESCRIPTION OF THE DRAWINGS

So that the matter in which the above-recited features, advantages and objects of the invention, as well as others which will become clear, are attained and can be understood in detail, more particular descriptions of the invention briefly summarized above may be had by reference to certain embodiments thereof which are illustrated in the appended drawings. These drawings form a part of the specification. It is to be noted, however, that the appended drawings illustrate preferred embodiments of the invention and therefore are not to be considered limiting in their scope.

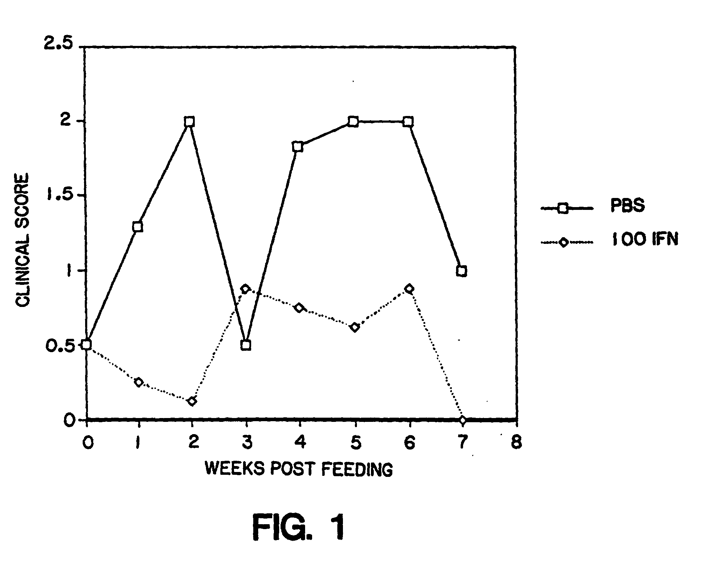

Figure 1 shows that orally administered murine natural IFN-α/β suppresses clinical relapse in murine CR-EAE. Two groups of six animals were inoculated and following the first attack were fed either mock IFN or 100 units murine natural IFN-α/β 3 times per week for seven weeks. One of two representative experiments is shown. SEM is < 10%.

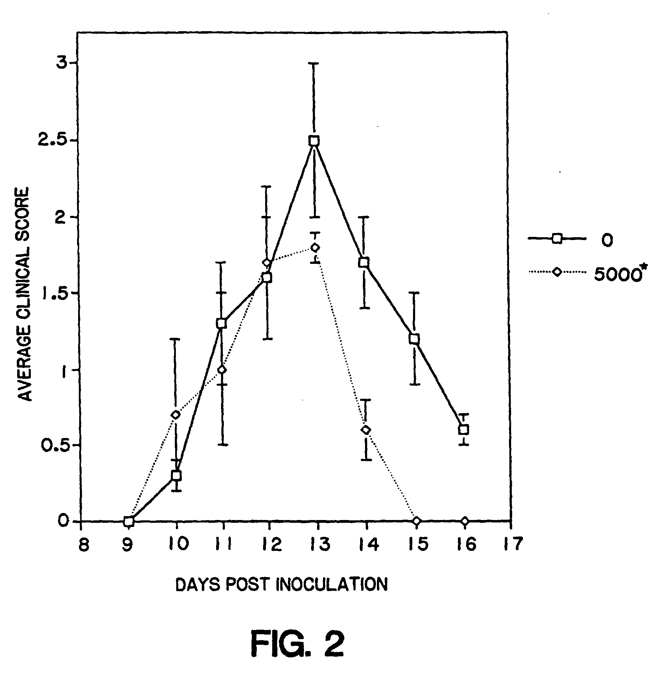

Figure 2 shows that oral administration of 5,000 units rat IFN-α/β inhibits clinical disease in acute EAE. Four groups of 6 Lewis rat were fed mock IFN, 1,000, 3,000 or 5, 000 units rat IFN-α/β for seven days preceding immunization [-7] and for 21 days thereafter [+14]. Results are expressed as average clinical score for each group on each day of disease post-inoculation ± SEM. One of two representative experiments is shown. Data for 1,000 and 3,000 units are not shown. *p < 0.02 between mock and 5,000 unit IFN-α/β fed animals.

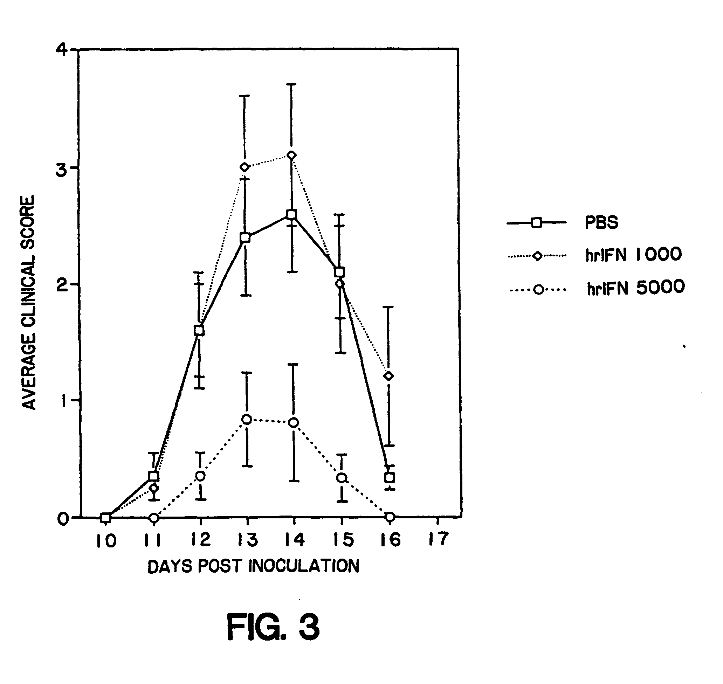

Figure 3 shows that oral administration of hrIFN-αa in rat acute EAE delays the onset, decreases the severity and speeds the recovery of clinical attacks. Three groups of seven Lewis rats were inoculated with MBP and CFA on day 0 and orally administered PBS, 1,000 units or 5,000 units hrIFN-α daily starting seven days preceding immunization [-7] and for 14 days thereafter [+14]. Values represent experimental allergic neuritis clinical scores for each group of 7 animals ± SEM. One of two representative experiments are shown.

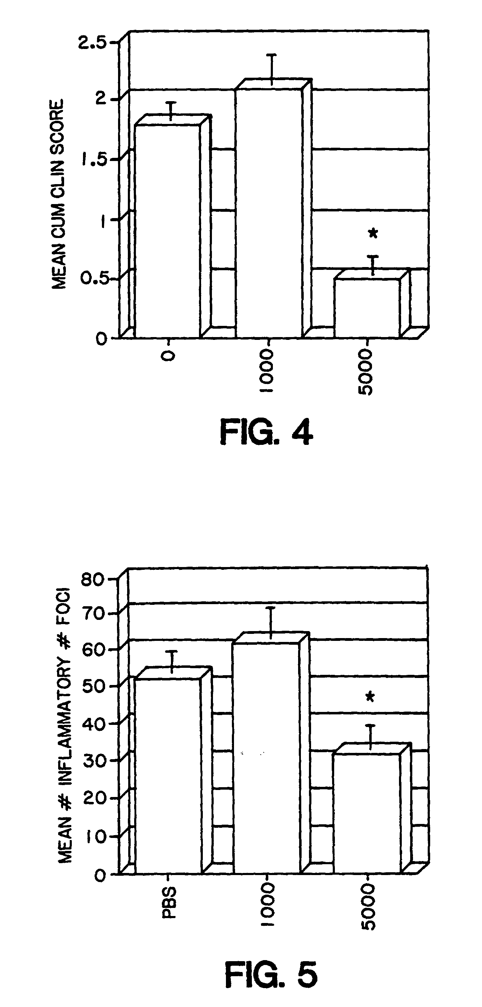

Figure 4 shows that oral administration of hrIFN-α in rat acute EAE decreases the experimental allergic neuritis cumulative clinical severity of acute clinical attacks. Three groups of seven Lewis rats were treated as described in Figure 3. Values represent experimental allergic neuritis cumulative clinical scores for each group of 7 animals from day 10 to 16 post-inoculation. *p < 0.001, 5,000 units IFN-α vs. both 1,000 units IFN-α and PBS control.

Figure 5 shows that oral administration of 5,000 units hrIFN-α decreases the number of inflammatory foci in spinal cord compared to the control and 1,000 interferon group. Following sacrifice, spinal cords from animals in Figure 3 were processed as described in methods. Results are expressed as # of inflammatory foci per cord ± SEM. One of two representative experiments is shown. *p < 0.04, 5,000 units IFN fed vs. 1,000 unit IFN fed or PBS control group.

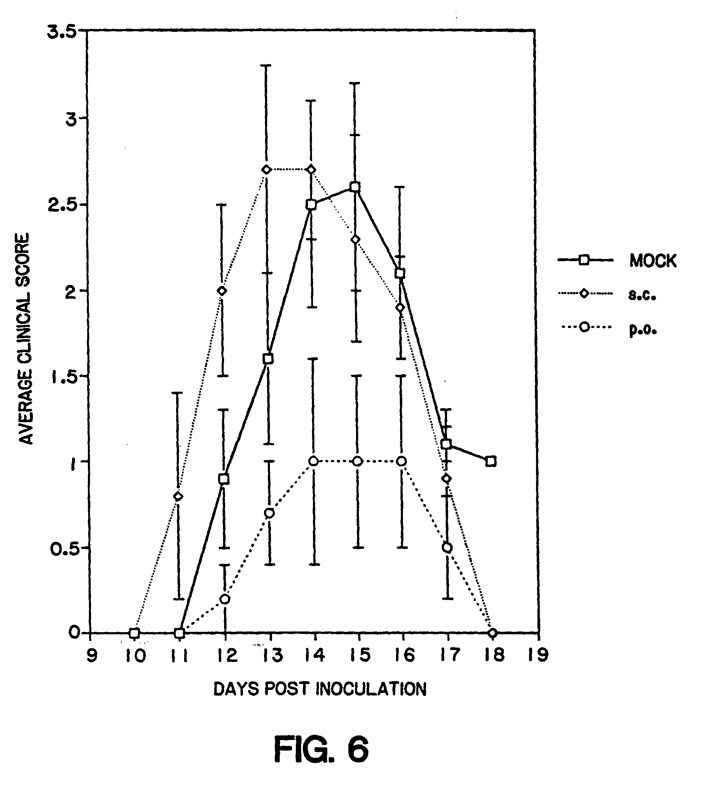

Figure 6 shows that oral administration of 5,000 units hrIFN-α, and not subcutaneous, administration decreases the severity of clinical attacks. Three groups of 6 Lewis rats were inoculated with MBP and CFA on day 0 and untreated, fed 5,000, or injected s.c. with 5,000 units hrIFN-α daily starting seven days preceding immunization [-7] and for 21 days thereafter [+14]. Values represent experimental allergic neuritis daily clinical scores for each group of 6 animals SEM. One of two representative experiments are shown.

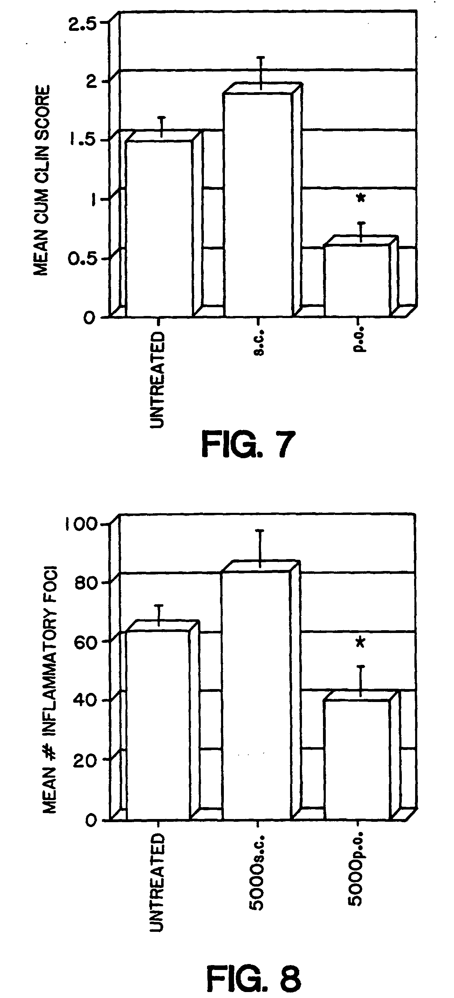

Figure 7 shows that oral administration of hrIFN-a in rat acute EAE decreases the experimental allergic neuritis cumulative clinical severity of acute clinical attacks. Three groups of six Lewis rats were treated as described in Figure 6. Values represent experimental allergic neuritis cumulative clinical scores for each group of 6 animals from day 10 to 16 post-inoculation. *p < 0.005, fed animals vs. untreated/s.c. treated animals.

Figure 8 shows that oral administration of 5,000 units hrIFN-α decreases the number of inflammatory foci in spinal cord compared to the control group. Following sacrifice, spinal cords from animals in Figure 6 were processed as described above and evaluated independently for foci of inflammation by one observer, without knowledge of the treatment status. Results are expressed as # of inflammatory foci per cord ± SEM. One of two representative experiments is shown. *p < 0.04, 5,000 units IFN fed vs. 5,000 units s.c. treated.

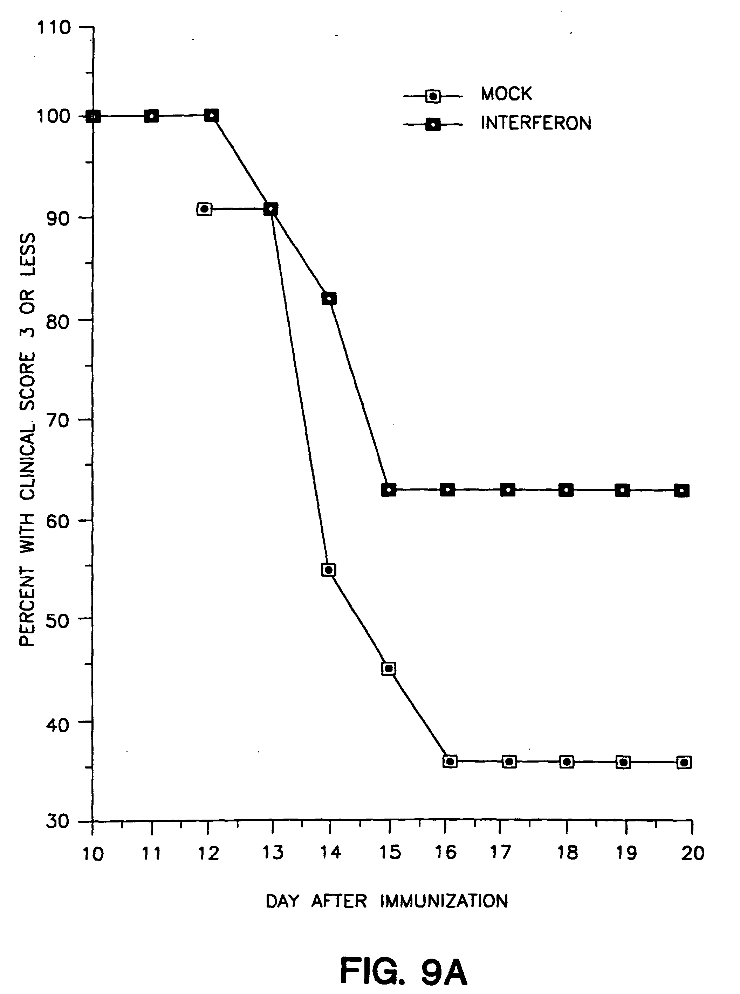

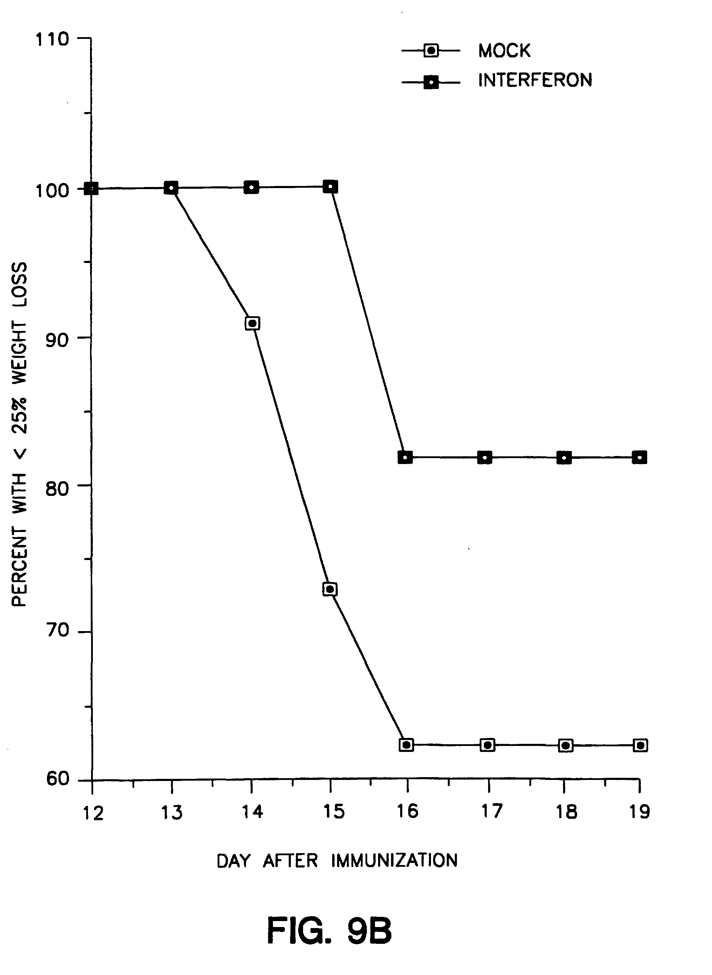

Figure 9A and 9B demonstrates that orally administered rat interferon-α/α reduces clinical disease in rats with experimental allergic neuritis. Lewis rats were fed either 5000 units IFN-α/β (interferon) or mock-IFN (mock) starting 7 days prior to immunization with bovine peripheral nerve myelin. Clinical score was significantly higher in the mock-IFN rats (n=11) than the IFN-α/β rats (n=11). More rats in the mock-IFN fed group than the IFN-α/β fed group reached a clinical score of 4 or higher (mock-IFN 7 of 11 vs IFN-α/β 4 of 11 rats) (Figure 9A) and lost 25% or more of their body weight (mock-IFN 4 of 11 vs IFN-α/β 2 of 11 rats) (Figure 9B).

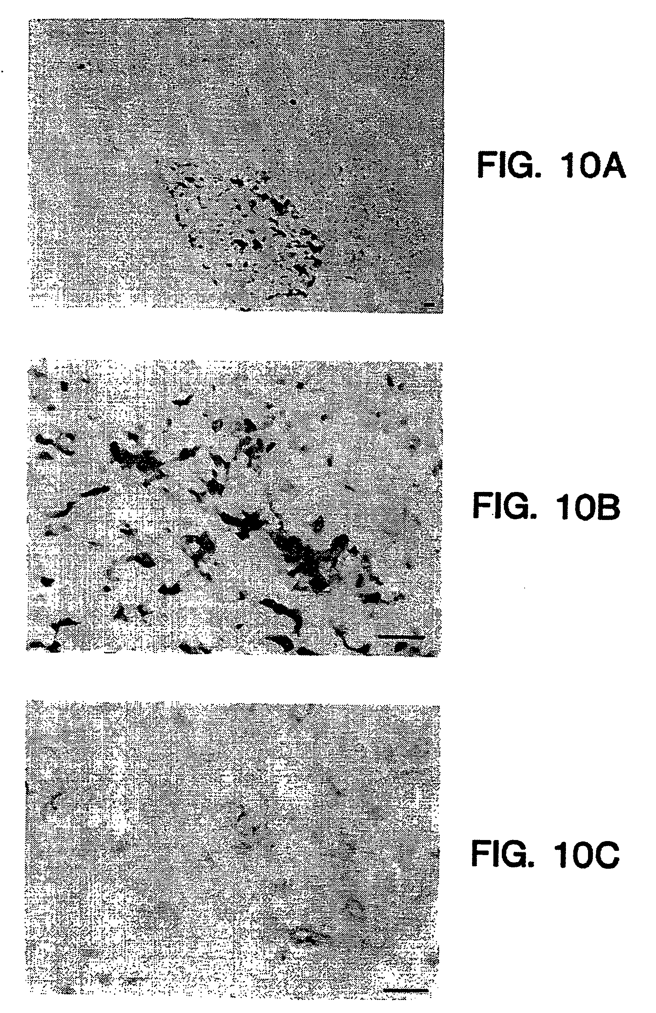

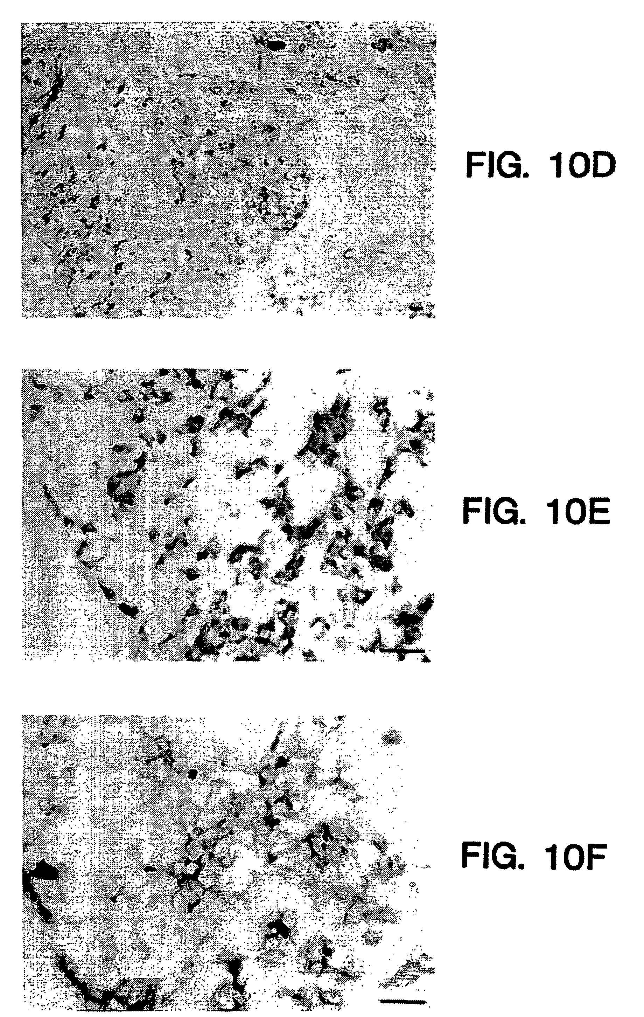

Figure 10 demonstrates that orally administered rat interferon-α/β reduced inflammation and IFN-γ production in experimental allergic neuritis. Lewis rats were fed either 5000 units IFN-α/β or mock-IFN starting 7 days prior to immunization with bovine peripheral nerve myelin until the time of sacrifice. Immunocytochemistry of lumbosacral nerve roots using ED1 antibody (1:1000) and IFN-γ antibody (1:10) on day 13 after immunization at the onset of clinical disease showed less ED1 positive macrophages (Figure 10A, 2B) in IFN-α/β fed rats compared to mock-IFN fed rats (Figure 10D, 10E). Concomitantly with reduced ED1 positive macrophages there was less IFN-γ expression in IFN-α/β fed rats (Figure 10C) compared to mock-IFN fed rats. (Figure 10F) Bar = 20 microns.

### DETAILED DESCRIPTION OF THE INVENTION

In the present invention, the modification of disease and inhibition of inflammatory cytokine production induced by orally administered cytokines in EAE provide a convenient, effective and long term means for treatment of autoimmune diseases of unknown antigen in humans, in particular multiple sclerosis.

The present invention is directed to the use of a type one interferon for the preparation of a medicament for the treatment of autoimmune disease wherein said medicament is to be administered orally. Generally, the interferon useful in the methods of the present invention is either alpha-interferon and beta-interferon. The type one interferon may be derived from any suitable source. Preferably, the interferon is selected from the group consisting of human recombinant interferon, rat interferon and murine interferon.

Generally, the type one interferon used in the present invention would be administered at a dose that effectively inhibits the onset or reoccurrence, etc., of the auto-immune disease. For example, the interferon may be administered in a dosage of from about 50 I.U./kg to about 25000 I.U./kg.

The uses of the present invention may be for any animal having an autoimmune disease. Most preferably, the uses of the present invention will be useful for humans.

Representative examples of auto-immune diseases include multiple sclerosis, rheumatoid arthritis, diabetes mellitus, psoriasis, organ-specific auto-immune diseases, chronic inflammatory demyelinating polyradiculoneuropathy and Guillain-Barré syndrome.

The use of the present invention is also for decreasing the severity or frequency of a relapse of multiple sclerosis in a human.

Moreover, the use of the present invention also is for inhibiting the onset of an auto-immune disease.

The following examples are given for the purpose of illustrating various embodiments of the invention and are not experimental allergic neuritis to limit the present invention in any fashion.

### EXAMPLE 1

### Induction of experimental autoimmune encephalomyelitis

A chronic relapsing form of EAE was induced in 7-10 week old female SJL/J mice using the method of Brown and McFarlin, Lab. Invest. (1981) 45:278-284 as modified by Miller et al., J. Immunol. (1987) 138:3776-3784. Briefly, each mouse received a subcutaneous injection over the shaved right flank of 0.3 ml of an emulsion containing 1 mg of syngeneic mouse spinal cord homogenate [MSCH] in 0.15 ml of phosphate buffered saline and 0.03 mg of *Mycobacterium* tuberculoses homines H37Ra [MT] [Difco Labs, Detroit, MI] in 0.15 ml of incomplete Freund's adjuvant [IFA]. Seven days later, the mice received a similar injection in the left flank. Initial clinical signs of disease were seen between days 13 and 25 postimmunization. Initial clinical signs of disease were seen between days 13 and 25 postimmunization. Clinical severity of the relapse attack was graded as follows by a blinded observer: 0 = no disease; 1 = minimal or mild hind limb weakness; 2 = moderate hind limb weakness or mild ataxia; 3 = moderate to severe hind limb weakness; 4 = severe hind limb weakness or moderate ataxia; 5 = paraplegia with no more than moderate four limb weakness; 6 = paraplegia with severe four limb weakness or severe ataxia. Animals were scored in a blinded fashion for 6-8 weeks and a cumulative weekly score was computed by averaging three scores per week [Monday, Wednesday, Friday] for each group of animals.

### EXAMPLE 2

### Cytokine feeding

Following initial attack, animals were fed varying doses [1-100 units] of murine natural IFNα/β [Cytimmune mouse IFN-α + β, 4.0 x 10⁵ IRU/ml, Lee Biomolecular Research, Inc., San Diego, CA], or mock murine IFN-α/β [Cytimmune < 2 IRU/ml, Lee Biomolecular Research, Inc., San Diego, CA. {generated identically to IFN-α/β except cultures are mock induced}] using a 2.5 cm syringe fitted with a 20 gauge ball point needle [Thomas Scientific, Swedesboro, NJ] three times per week [Monday, Wednesday, Friday] for 6-8 weeks. Mock IFN were used as control since heat denatured IFN may retain some immunological activity.

### EXAMPLE 3

### Preparation of Guinea Pig Myelin Basic Protein [GP-MBP]

Twenty grams of guinea pig spinal cord were added to 4.7 ml -20°C methanol and homogenized with a tissue homogenizer. 9.4 ml -20°C chloroform was added, homogenized an additional 2 minutes and stirred for 60 minutes at room temperature; then at 4°C for an additional 60 minutes. The material was filtered through 2 thicknesses of sterile gauze through a chilled Buchner flask and a #1 Whatman filter paper. The tissue cake was rinsed with 20 ml -20°C acetone and was resuspended in 40 ml of cold ddH₂O; then stirred 30 minutes at 4°C, filtered a second time and resuspended in 10 ml of cold ddH₂O. Suspension was sonicated to facilitate resuspension of pellet in ddH₂O.

Tissue/H₂O slurry was adjusted to pH = 3 with 1 N HCl and stirred overnight at 4°C. After an overnight spin, pH was readjusted to 3 and spun at 10,000 x g for 15 minutes at 4°C. Supernatant was filtered through #1 Whatman paper and liquid filtrate retained. Filtrate was adjusted to pH = 9 with 10 N NaOH and stirred for 60 minutes at 4°C; then spun at 10,000 x g for 15 minutes at 4°C. Supernatant volume was measured and ammonium sulfate added to a final concentration of 27.7 grams/100 ml; supernatant was stirred in cold for 30 minutes and then spun at 10,000 x g for 15 minutes at 4°C and resuspended in 1 ml ddH₂O. Resuspended pellet was dialyzed in small molecular weight [6-8000 MW] dialysis tubing against ddH₂O. Protein concentration was determined at A₂₈₀ via UV spectroscopy. To check purity of myelin basic protein preparation, a 7-20% SDS-PAGE gradient gel was prepared and stained with 0.3 M CuCl₂. Purity of guinea pig myelin basic protein [GP-MBP] preparation was demonstrated by an 18 kD band.

### EXAMPLE 4

### Lymph Node Cell and Spleen Preparation

Animals were killed 6-8 weeks after initiation of feeding and draining inguinal nodes and spleen cells were removed and single cell suspensions were made through a 90 um stainless wire meshes. Red cell lysis was performed in the spleen cell suspensions with 2 mls of ACK solution added to the pellet and the reaction allowed to continue at 5 minutes at room temperature.

### EXAMPLE 5

### In vitro T cell proliferation

Seven days following clinical relapse attack, mice were sacrificed, draining inguinal lymph nodes were pooled and cultured in vitro to determine antigen-specific T cell proliferative responses. Antigen stimulation was carried out with antigen at either 0 or 100 µg/ml [GP-MBP or MT] and mitogen stimulation with Con A at 2.5 µg/ml by incubating lymph node cells at 2 x 10⁵ cells/well in RPMI [Gibco, Grand Island, NY] supplemented with 10% fetal calf serum [FCS] [Whittaker Bioproducts, Walkersville, MD], 1% sodium pyruvate [Gibco, Grand Island, NY], 1% glutamine [Gibco], 1% Penicillin/Streptomycin, and 50 µM 2-mercaptoethanol. The plates were incubated at 5% CO₂ and humidified at 37°C for 4 days. At that time the cells were pulsed with 2 µCi of tritiated [³H] dTd and harvested 18 hours later on an automated harvester. [³H] dTd uptake was measured in a Beckman [liquid] scintillation counter. Cultures were run in triplicate and the results expressed as ΔCPM.

### EXAMPLE 6

### Histology

Following sacrifice, spinal cords were removed and immersion fixed in 10% neutral buffered formalin for a minimum of two weeks. After fixation, brains were bisected in the horizontal plane, and cords sectioned in entirety in the horizontal plane at approximately 3 mm intervals and processed to paraffin. Paraffin blocks were sectioned at 6-8 microns, and step sections were stained with hematoxylin and eosin and with Luxol-fast blue/PAS/hematoxylin and examined by light microscopy. Cord sections were evaluated independently for foci of inflammation by a blinded observer, without knowledge of the treatment status of the animals prior to sacrifice. Spinal cord tissue was sampled in an identical fashion for each animal and numbers of inflammatory foci per section [> 20 inflammatory cells] in the parenchyma were counted.

### EXAMPLE 7

### Cytokine analysis

Spleen cells from mock and 100 unit murine IFN-α/β treated animals were cultured with Con A [2.5 ug/ml] at 1 x 10⁶ cells /ml in 75 cm² tissue culture flasks for 48 hours in a humidified 5% CO₂/95% air incubator at 37°C. Supernatants was collected at 24 and 48 hours after Con A activation and frozen at -70°C after centrifugation. Interleukins was measured using solid phase ELISA assay. Anti-IL-2, anti-IL-10, or anti-IFN-γ [PharMingen, San Diego, CA] was incubated on polyvinyl plastic 96 well microtiter plates with 0.01M carbonate buffer (pH 9.6) overnight at 4°C. The plates were blocked with 3% BSA in phosphate buffered saline for 3 hours. 100 µl of supernatants was added at various dilutions that are tittered to the linear portion of the absorbance/concentration curve in triplicate and incubated for 1 hour at room temperature. After the plates was washed five times with phosphate buffered saline Tween (0.05%), 100 µl peroxidase conjugated interleukin monoclonal antibody (with a different epitopic determinant than the first antibody used to coat the polyvinyl plate) at a 1:1000 concentration was added for 60 minutes. Subsequently, the peroxidase substrate o-phenylenediamine dihydrochloride was added and the absorbance measured at 450 nm. Standard curves with various amounts of the different interleukins were generated. Statistical analysis was performed using a one-tailed Student's t-test.

### EXAMPLE 8

### Orally administered murine type I interferons suppress clinical disease, inflammation and inhibit proliferative responses to MBP

One to ten units of orally administered type 1 IFN had an immunological effect but was not adequate to suppress clinical relapses. Therefore, two groups of six immunized SJL/J mice were fed mock IFN or 100 units murine natural IFN-α/β three times per week beginning on day 30, following recovery from the first attack. Group clinical scores after the initial attack were not significantly different among the different groups. Clinical relapses occurred approximately 40 days after inoculation. Clinical scores demonstrated significant differences in outcome in the mock IFN fed versus 100 units murine natural IFN-α/β fed animals [p < 0.03, see Figure 1]. Two major relapses occurred in the mock IFN fed group over the course of seven weeks with a resultant increased neurological deficit. The oral murine natural IFN-α/β group underwent a delayed single attack without residual neurological deficit. Oral murine natural IFN-α/β blunted the severity and decreased the group score between the initial attack and relapse.

Con A activation of draining inguinal lymph node cells was inhibited in mock IFN fed compared to animals fed 100 units murine natural IFN-α/β [88,222 cpm ± 1,910 vs. 38,095 cpm ± 3,160]. Lymph node cells from mock IFN fed animals tested with MBP generated a robust proliferative response, but that response was profoundly inhibited in murine natural IFN-α/β fed animals [14,052 cpm ± 842 vs. 448 cpm ± 50].

To determine whether there was inhibition to another sensitized antigen, antigen-specific proliferation of draining inguinal lymph node to a second sensitized antigen was examined, *Mycobacterium tuberculoses homines* [MT], a component of MSCH inoculum. Lymph node cells from mock IFN fed animals generated a robust proliferative response to MT but that response is profoundly decreased in 100 unit murine natural IFN-α/β fed animals [52,401 cpm ± 857 vs. 5,214 cpm ± 808].

Animals were also examined histologically 65 days following immunization. There were significantly less inflammatory foci in the IFN fed group [0.5 ± 0.1] compared to the control mock IFN group [1.8 ± 1.1] [p < 0.05]. Thus, orally administered murine natural type I interferons can suppress clinical disease, decrease inflammation and inhibit proliferation to MBP and MT.

### EXAMPLE 9

### Suppression of relapse by oral IFN-α/β correlates with decreased IFN-γ secretion

The intensity of disease in EAE has been associated with IFN-γ secretion after Con A stimulation of spleen cells. Therefore pooled spleen cells were stimulated from both mock fed [n=5] or 100 units fed [n=5] murine IFN-α/β with Con A [2.5 µg/ml] at 1 x 10⁶ cells /ml for two days and supernatants were assayed by solid phase ELISA [IL-2, IL-10, IFN-γ]. Oral IFN-α/β consistently decreased IFN-γ a mediator of inflammation [exp #1, mock spleen 2,500 ng/ml ± 300 vs 100 unit spleen *1,100 ng/ml ± 200; exp #2, mock spleen 2,180 ng/ml ± 100 unit spleen 247 vs *143 ng/ml ± 13, * p < 0.001 compared to mock IFN control]. There was also a decrease in IL-2, a T cell growth factor and increased IL-10 production, although these changes did not attain statistical significance.

The present invention illustrates that orally administered type 1 interferons can modify the biological response in CR-EAE when administered after animals recover from the initial attack. When given at adequate dosages, orally administered type 1 interferons suppress clinical relapses, diminish inflammation and inhibit proliferation of activated cells from draining inguinal lymph nodes, the natural sites for high frequencies of activated T cells from subcutaneously administered antigens. Thus, type 1 interferons are active by the oral route with significant immunological effects in susceptible immune compartments. Oral IFN consistently decreased Con A induced IFN-γ secretion in spleen cells. Suppression of proliferation to MBP can occur even after a period of 6 weeks post-inoculation and 4 weeks after initial clinical attack. Thus, orally administered cytokines, lacking a protective matrix to prevent protein digestion in the oropharynx and the rest of the alimentary canal, are capable of inhibiting proliferation to previously sensitized antigens.

The blunting of an established and ongoing immune response is an important therapeutic issue. The administration of animal myelin antigens by the oral route can improve the clinical course of CR-EAE in rats and guinea pigs and decrease pathological sequelae to myelin antigens in CR-EAE. Oral administration of myelin proteins can decrease the severity of attacks in male DR2- MS patients and the frequency of MBP specific T cell lines in myelin treated individuals. However, in the human trials and animal experiments, there was only partial suppression of clinical or pathological disease suggesting that other orally administered immunoactive substances may be superior to myelin antigens.

The immunomodulatory mechanism of orally administered type 1 interferon is not known. Modulatory effects of Con A activated lymphocytes on the mitogen responses of normal responder cells can be abrogated by addition of anti-human leukocyte IFN serum *in vitro;* this may prevent the production of inhibitory factors induced by IFN-α, e.g. soluble immune response suppressor [SIRS] and macrophage derived suppressor factor [MØ-SF]. Peripheralized T cells may be required for interferon production after such mitogen stimulation. However, oral administration of low dose IFN-α in mice does not result in detectable levels of IFN-α in the blood in contrast to intraperitoneal administration, nor can its effect be blocked by circulating anti-IFN antibodies. The neutropenic effect of orally administered IFN can be transferred by injection of blood cells but not serum to recipient animals. Cytokine mRNA analysis of the CNS in EAE suggests that IL-2, IL-6 and IFN-γ are elevated in acute disease but during stabilization of symptoms these cytokines are decreased with increasing IL-10 levels which can regulate Th1 cells. Therefore type 1 IFN may be an immunomodulatory molecule which induce suppressor factors, such as IL-10, inhibiting response to immunized antigens such as MBP and MT.

In summary, the oral administration of biological response modifiers such as type I interferons provides a potentially non-toxic, convenient and continuous means of inhibiting immune responsiveness. Biological response modifiers delivered by the oral route provide another means to treat autoimmune diseases.

### EXAMPLE 10

### Induction of acute experimental autoimmune encephalomyelitis

EAE was induced in 8-10 week old female Lewis rats [Harlan Sprague Dawley, Indianapolis, IN], using an emulsion containing equal parts of myelin basic protein in PBS and complete Freund's adjuvant [CFA]; *Mycobacterium tuberculoses* homines H37Ra [MT] [Difco Labs, Detroit, MI]. Lewis rats were immunized s.c. in a rear footpad with 0.1 ml emulsion containing 50 µg MBP and 500 µg MT in Freund's adjuvant. Initial clinical signs of disease were seen between days 9 and 11 postimmunization. Clinical severity of the initial attack was graded as follows by a blinded observer: 0 = no disease; 1 = flaccid tail; 2 = slight hind limb weakness; 3 = moderate hind limb weakness; 4 = total hind limb weakness and incontinence. Average daily clinical score was calculated by determining the average score for each group on each day of attack. A experimental allergic neuritis cumulative clinical score was calculated that incorporated overall clinical severity, disease incidence and duration of clinical signs into one comparative index by summing all daily scores on all rats in a treatment group between onset and end of attack and dividing by the total number of rats within the treatment group. Statistical analysis was performed on combined data from at least two similar experiments.

### EXAMPLE 11

### Administration of IFN

Seven days preceding immunization [-7] and for 21 days thereafter [+14], rats were fed either mock IFN, 1,000, 3,000 or 5,000 units rat natural IFN-α/β [Cytimmune rat IFN α + β, 4.0 x 10⁵ IRU/ml, Lee Biomolecular Research, Inc. San Diego, CA] or human recombinant IFN-α [hrIFN-α] [IFN-α₁₁ 3.0 x 10⁶ IU/ml, Schering Corp, Kenilworth, NJ] using a syringe fitted with a 20 gauge ball point needle [Thomas Scientific, Swedesboro, NJ] placed in the posterior oropharynx or injected subcutaneously with PBS placebo or 5,000 units hrIFN-α daily. Lyophilized IFN preparations were reconstituted with DPBS and diluted 1/600 to 1/3,000. The mock natural preparation was prepared identically to the rat natural IFN-α/β but is not induced with Newcastle virus.

Preparation of guinea pig myelin basic protein [GP-MBP], i.e., homogenization and dilapidation, acid elution of MBP, lymph node cell and spleen preparation, in vitro T cell proliferation and cytokine analysis were performed as described above.

### EXAMPLE 12

### Histology

Following sacrifice, spinal cords were removed and immersion fixed in 10% neutral buffered formalin for a minimum of two weeks. After fixation, cords were sectioned in entirety in the horizontal plane at approximately 3 mm intervals and processed to paraffin. Paraffin blocks were sectioned at 6-8 microns, and step sections [n=5] were stained with hematoxylin and eosin and with Luxol-fast blue/PAS/hematoxylin, and examined by light microscopy. Cord sections were evaluated independently for foci of inflammation by a blinded observer, without knowledge of the treatment status of the animals prior to sacrifice. Spinal cord tissue was sampled in an identical fashion for each animal and numbers of inflammatory foci per section (>20 inflammatory cells) in the parenchyma were counted. Statistical analysis was performed using a two-tailed Student's t-test.

### EXAMPLE 13

### Natural rat interferon alpha/beta modifies clinical disease, inhibits proliferative response and decreases inflammation to GP-MBP

Four groups of six 8-10 week old Lewis rats were immunized with equal parts MBP and CFA and subsequently had attacks beginning by day 9 and followed until day 16. Seven days preceding immunization [-7] and for 21 days thereafter [+14], each group of animals were fed either mock IFN, 1,000, 3,000, or 5,000 units rat type 1 IFN-α/β daily in 0.1 ml PBS. All animals were scored for clinical disease until day 16 after immunization. Results from blinded examination of daily group clinical scores demonstrated significant differences in clinical outcome in the mock fed versus 5,000 units IFN-α/β fed animals particularly at days 13-16 but not in the animals fed 1,000 or 3,000 units. Rats treated with 5,000 units rat natural IFN-α/β had peak disease that was less severe than in the mock group, and the 5,000 unit IFN-α/β treated group recovered more quickly and returned to baseline sooner than the mock treated group [Figure 2, data not shown for 1,000 and 3,000 units]. The experimental allergic neuritis cumulative clinical score of the animals treated with 5,000 units rat natural IFN-α was significantly less than mock IFN fed controls [0.8 ± 0.2, 5,000 unit fed vs 1.2 ± 0.2, mock IFN fed, p < 0.02]. Animals were also examined histologically 16 days following immunization. There were less inflammatory foci in the IFN-α/β treated [26 ± 6] compared to the control mock interferon group [50 ± 2] although this did not attain statistical significance due to the small number of spinal cords examined per group [n=3] [p < 0.06].

Draining popliteal lymph node Con A proliferation was inhibited from 16,209 ± 1,234 cpm from mock fed animals to 8,120 ± 765 cpm in 5,000 unit IFN-α/β fed animals. Draining popliteal lymph node cells from mock and 5,000 unit treated animals were stimulated with ionomycin + PMA and demonstrated decreasing proliferation from mock [20,505 cpm ± 505] to 5,000 [6,111 cpm ± 636] unit treated animals. No consistent differences in MBP or MT proliferation between fed and mock fed animals was demonstrated in draining popliteal lymph node. There was also no inhibition in spleen or non-draining mesenteric lymph node to Con A or ionomycin/PMA in IFN treated animals.

Thus, species specific type I interferons inhibit the severity of acute clinical disease when given at adequate dosages. Inhibition of proliferation from orally administered IFN-α/β could be due to a direct action of IFN which enters the bloodstream through the gut or indirectly. The *in vitro* type I IFN treatment of draining popliteal lymph node and spleen cells was examined from immunized but mock treated animals demonstrates similar effects to *in vivo* treatment. There was no clear effect on Con A proliferation in draining popliteal lymph node or spleen cells exposed to IFN [1-100 units] *in vitro,* in contrast to *in vivo* IFN oral administration in which draining popliteal lymph node proliferation was decreased. Thus, an indirect effect of type 1 IFN may be mediated through the gut associated lymphoid tissue.

### EXAMPLE 14

### Modification of acute rat EAE by oral administration of human recombinant interferon alpha

Since human type 1 interferon can show cross-species activity in mice, guinea pigs, gnotobiotic calves, horses and pigs, and cats, recombinant human interferon [hrIFN-α], a uniform material that may provide more immunosuppression per unit of activity than natural preparations, was utilized. For seven days preceding immunization [-7] and for 21 days thereafter [+14], each group of animals was fed either PBS, 1,000 units or 5,000 units hrIFN-α daily. Three groups of seven 8-10 week old Lewis rats were immunized and subsequently had an attack beginning by day 10 and extending through day 16. All animals were scored for clinical disease until day 16 after immunization. As shown in Figure 3, PBS and 1,000 unit hrIFN-α fed animals demonstrated severe acute attacks starting at day 10 and reaching peak intensity at day 14. Animals fed 5,000 units hrIFN-α showed delayed attack onset, decreased severity at peak and earlier resolution of the attack. The experimental allergic neuritis cumulative clinical score of the animals treated with 5,000 units hrIFN-α was significantly less than either PBS control fed or 1,000 unit fed animals [Figure 4]. Thus, orally administered hrIFN-α suppress the onset of clinical attacks of EAE in the Lewis rat when administered before inoculation. Following sacrifice 16 days post-immunization, animals were also examined histologically. There were significantly fewer inflammatory foci in 5,000 unit hrIFN-α fed group compared to the PBS control group or 1,000 unit hrIFN-α fed group [Figure 5].

### EXAMPLE 15

### Oral but not equivalent doses of parenteral administered human recombinant interferon alpha modifies clinical disease

Orally administered hrIFN-α modifies clinical disease and decrease inflammation in spinal cord. The relative efficacy of equivalent amounts of orally versus parenterally administered hrIFN-α was examined. Three groups of six Lewis rats were immunized and either untreated, fed 5,000 units hrIFN-α or injected with 5,000 units hrIFN-α s.c. for seven days preceding immunization [-7] and for 21 days thereafter [+14]. All animals were scored for clinical disease until day 18 after immunization. Rats treated with oral 5,000 units hrIFN-α had a less severe disease at peak of the disease and more rapid recovery compared to the untreated group [Figure 6]. Indeed, the untreated and the s.c. treated groups had very similar clinical curve scores, suggesting that s.c. hrIFN-α had little or no effect on clinical disease. Experimental allergic neuritis cumulative clinical scores demonstrated significant differences in clinical outcome in the untreated/sc treated vs fed animals [Figure 7]. There was no significant difference between untreated and s.c. treated animals. Eighteen days following immunization, animals were sacrificed and examined histologically. There were significantly fewer inflammatory foci in 5,000 unit fed compared to the 5,000 units s.c. treated group [Figure 8].

PBS fed and PBS s.c. injected controls demonstrated similar findings. In this case four groups of six Lewis rats were either fed PBS, injected s.c. with PBS, fed 5,000 units hrIFN-α or injected with 5,000 units hrIFN-α s. c. for seven days preceding immunization [-7] and for 21 days thereafter [+14]. All animals were immunized on day 0. Animals were scored for clinical disease until day 16 after immunization and sacrificed. Experimental allergic neuritis cumulative clinical scores were significantly less in hrIFN-α fed animals [1.0 ± 0.2] compared to PBS fed [2.5 ± 0.4] [p < 0.005]. There was no significant difference between mock s.c. [1.7 ± 0.4] and 5,000 units hrIFN-α s.c. [2.1 ± 0.4] animals even though s.c. treated animals did have higher experimental allergic neuritis cumulative clinical scores. Thus, s.c. administered hrIFN-α cannot modify the onset of acute clinical disease when given at clinically preventive oral dosages.

### EXAMPLE 16

### Orally administered IFN-α inhibits the mitogen induced production of IFN-γ in draining popliteal lymph nodes

The intensity of disease in EAE has been associated with TFN-γ secretion after Con A stimulation. Therefore, spleen and draining popliteal lymph node cells [day 18 post-immunization] from both mock fed or 5, 000 units fed hrIFN-α rats were stimulated with Con A [2.5 µg/ml] for two days and supernatants were assayed by ELISA. The results in TABLE I show that oral hrIFN-α decreased IFN-γ, a mediator of inflammation, in draining popliteal lymph node but not in spleen.

**TABLE I**

| Inhibition of clinical disease by oral IFN correlates with decreased IFN-γ secretion in draining popliteal lymph nodes | | | | |
|---|---|---|---|---|
| | LN mock | LN 5,000 | spleen mock | spleen 5,000 |
| IFN-γ [ng/ml] | 461 ± 60 | 96 ± 32* | 360 ± 75 | 310 ± 62 |

| | | | | |
|---|---|---|---|---|
| Spleen and draining popliteal lymph node cells [day 18] from mock IFN and 5,000 unit IFN fed immunized rats were cultured with Con A [2.5 µg/ml] at 1 x 10⁶ cells/ml for 48 hours. Supernatants were collected at 48 hours after Con A activation and frozen at - 70° C after centrifugation. IFN-γ was measured using solid phase ELISA assay. Results given are expressed in ng/ml. Combined data from two experiments are shown. LN 5,000 compared to LN mock *p < 0.05. | | | | |

Orally administered type 1 interferons, as opposed to identical s.c. doses, are disclosed by the present invention as modifiers of biological response to MBP in acute EAE in Lewis rats when they are administered before sensitization and clinical attack. Orally administered type 1 interferons partially modify clinical attacks, decrease the number of inflammatory foci in spinal cord, decrease non-specific proliferation by Con A and ionomycin/PMA and decrease the production of IFN-γ in draining popliteal lymph nodes. This suggests that IFN-α is more active by the oral route, and has definable immunological effects, and confirms that specific cytokines are capable of inhibiting clinical disease when given via the GI tract.

Surprisingly, both human recombinant IFN-α and species-specific rat natural IFN worked in present invention. Natural IFNs are a mixture of 14 separate subspecies including the IFN-α₁₁ subtype which may be only a small component of the natural type. Human IFN shows some cross species activity, however, the exclusive use of the IFN α₁₁ subtype may provide a relative greater amount by inhibitory activity/total units of activity of the most important component for immunosuppression in the rat.

Antiproliferative effects of orally administered IFN were greater in draining popliteal lymph node than in non-draining popliteal lymph nodes. The oral administration of IFN-α also inhibited the production of IFN-γ in draining popliteal lymph nodes. Draining popliteal lymph node are the natural draining areas for subcutaneously administered antigens and therefore presumably the reservoir of high frequencies of sensitized MBP-specific T cells. Orally administered cytokines may preferentially effect proliferation and cytokine production at sites of immune activation compared to systemic administration. Inhibition of IFN-γ secretion in an activated regional immune compartment by IFN-α may cause decreased inflammatory effect of MBP-specific cells in the CNS.

Oral IFN-α was effective in inhibiting EAE compared to identical s.c. doses. *In vitro* IFN did not inhibit Con A proliferation in pop LN in contrast to *in vivo* p.o. administration. Thus, the route of administration is critical and may determine the immunological mechanism for IFN. Proteins which might not survive transit through the alimentary canal may still exhibit immunoinhibitory activity via the gut associated lymphoid tissue [GALT] in the oropharynx. Therefore oral administration provides an alternative drug delivery system for therapeutic cytokines in the treatment of autoimmune diseases by the generation of immunoregulatory cells via the gut immune system.

The immunomodulatory mechanism of orally administered type 1 interferon is not known. Modulatory effects of Con A activated lymphocytes on the mitogen responses of normal responder cells can be abrogated by addition of anti-human leukocyte IFN serum *in vitro* which may modify the production of inhibitory factors induced by IFN-α, e.g. soluble immune response suppressor [SIRS] and macrophage derived suppressor factor [MØ-SF]¹⁶. Peripheralized T cells may be required for interferon production after such mitogen stimulation. However, oral administration of low dose IFN in mice does not result in detectable levels of IFN in the blood in contrast to intraperitoneal administration, nor can its effect be blocked by circulating anti-IFN antibodies. The neutropenic effect of orally administered IFN can be transferred by injection of blood cells but not serum to recipient animals. Similarly, cells can be induced in Peyer's patch after oral administration of antigen in mice that decrease humoral response *in vitro.* Therefore, type 1 IFN may be an immunomodulatory molecule produced by activated T and other immune cells which induce suppressor factors, such as SIRS and MØ-SF, inhibiting response to immunized antigens such as MBP.

The present invention demonstrates that oral IFN-α therapy is relevant for relapsing-remitting multiple sclerosis and other chronic non-neurological autoimmune diseases since orally administered IFN-α appears to be more effective than equivalent doses of parenteral IFN-α. Thus, the oral administration of cytokine biological response modifiers such as IFN-α provides an effective means of modifying clinical attacks to an autoantigen when administered before sensitization. The oral route is a convenient drug delivery system that allows the use of significantly lower doses of cytokines, minimizing side effects and enhancing efficacy.

### EXAMPLE 17

### Immunization

Female Lewis rats (Harlan), 150-170 g in weight, were immunized with 20 mg (wet weight) peripheral nerve myelin emulsified in an equal volume of complete Freund's adjuvant (CFA) which contained 10 mg *M.tuberculosis* per ml Freund's. The immunogen was injected in 200 µl into the footpad of the right hindleg. Peripheral nerve myelin was isolated from bovine spinal roots (Pel-Freeze, TX) according to the method of Norton, Methods Enzymol., 31 (part A), 435, 1975.

### EXAMPLE 18

### Interferon administration

Starting seven days preceding immunization until sacrifice at 20 days after immunization, rats were fed either mock-IFN or 5000 U rat natural IFN-α/β (Cytimmune rat IFN-α/β 1.5 x 10⁶ U/ml, Lee Biomolecular Research Inc, San Diego, CA). Lyophilized IFN was reconstituted with sterile water, diluted in phosphate buffered saline (PBS) and administered in a 0.1 ml daily dose using a syringe fitted with a 20 gauge ball point needle (Thomas Scientific, Swedesboro, NJ) placed in the posterior oropharynx.

### EXAMPLE 19

### Clinical Assessment

The rats were weighed daily starting at day 7 after immunization and evaluated clinically using the following scale: 0, normal; 1, limp tail; 2, abnormal gait; 3, mild paraparesis; 4, severe paraparesis; 5, paraplegia; 6, paraplegia with forelimb involvement; 7, paraplegia with forelimb involvement and respiratory distress; 8, moribund or dead as described by Hahn et al., Acta Neuropathol. 82:60-65 (1991). Weight loss was calculated for each animal as the difference between weight at time of sacrifice and maximum weight and expressed as a percentage of the maximum weight.

### EXAMPLE 20

### Proliferation assays

At sacrifice on day 20 after immunization, draining popliteal lymph nodes and spleen were removed. Single cell suspensions were made by passage through 90 µm stainless wire meshes. Red cell lysis was performed in spleen cell suspensions by adding 2 ml of ACK solution to the pellet. Cells from spleens and draining lymph nodes were pooled for each treatment group for *in vitro* culture. Whole spleen or lymph node populations were incubated at 2 x 10⁵ cells/ml with *M.tuberculosis* at 10 µg/ml, SP26 at 1 µg/ml [SP26 is a neurotogenic peptide of 26 aminoacids (Rostami et al., J. Neuroimmunol., 30:145-151 (1990) synthesized in the Department of Analytical Chemistry at the University of Texas], Concanavalin A at 2.5 µg/ml (Sigma Chemical Co., St.Louis, MO) in RPMI (Gibco, Grand Island, NY), ionomycin at 100 ng/ml (Calbiochem, La Jolla, CA) in combination with myristic acid palmityl ester (PMA) at 1 ng/ml (Sigma Chemical Co., St.Louis, MO) supplemented with 10% fetal calf serum (FCS; Whittaker Bioproducts, Walkersville, MD), 1 mM sodium pyruvate (Gibco, Grand Island, NY), 2 mM glutamine (Gibco), 100 units Penicillin/Streptomycin, and 50 µM 2-mercaptoethanol. The plates were incubated at 5% CO₂ and humidified at 37°C for 4 days. At that time, the cells were pulsed with 2 µCi of tritiated (³H) dTd and harvested 18 hours later on an automated harvester. (³H) dTd uptake was measured in a Beckman liquid scintillation counter. Cultures were run in triplicate and the results expressed as ΔCPM.

### EXAMPLE 21

### Cytokine analysis

Draining pooled popliteal lymph node and pooled spleen cells from mock and 5000 unit rat IFN treated experimental allergic neuritis animals were cultured with Con A (2.5 µg/ml), *M.tuberculosis* (10 µg/ml) or SP26 (1 µg/ml) at 1 x 10⁶ cells/ml in 75 cm² tissue culture flasks for 48 hours in a humidified 5% CO₂/95% air incubator at 37°C. Supernatants were collected after centrifugation and frozen at -70°C. IFN-γ was measured using a solid phase ELISA assay. Anti-IFN-γ (Pharmingen, San Diego, CA) was incubated on polyvinyl plastic 96 well microtiter plates with 0.01M carbonate buffer (pH 9.6) overnight at 4°C. The plates were blocked with 3% bovine serum albumin (BSA) in PBS for 3 hours. One hundred µl of supernatants were added at various dilutions tittered to the linear portion of the absorbance/concentration curve in triplicate and incubated for 1 hour at room temperature. After the plate was washed 5 times with PBS Tween (0.05%), 100 µl peroxidase conjugated IFN-γ monoclonal antibody (with a different epitopic determinant than the first antibody used to coat the polyvinyl plate) at a 1:1000 concentration was added for 60 minutes. Subsequently, the peroxidase substrate O-phenylenediamine dihydrochloride was added, and the absorbance measured at 450 nm. Standard curves with various amounts of IFN-γ were generated.

### EXAMPLE 22

### Histology

Rats were sacrificed by perfusion with saline under pentobarbital anesthesia on day 20 after immunization. The cauda equine was removed and divided. The distal portion was fixed in 2.5% paraformaldehyde/2.5% glutaraldehyde in PBS for 4-6 hours, osmicated overnight in 2% osmium tetroxide, dehydrated and embedded in Epon. Four to five blocks of the lower lumbosacral roots were prepared from each animal. Representative 0.5 micron sections of each block were stained with toluidine blue.

Histological assessment of demyelination and inflammation in each root was performed using the following scale (Hahn et al, 1991): demyelination 1+ = a few demyelinated axons perivenular or scattered; 2+ = many foci of perivenular demyelination; 3+ = extensive demyelination perivenular and confluent; inflammation 1+ = a few scattered mononuclear inflammatory cells often subperineurial; 2+ = perivenular cuffing with mononuclear inflammatory cells; 3+ = extensive multifocal perivenular cuffing and widespread endoneurial inflammation. Scores were calculated per animal by dividing the total number for demyelination and inflammation over the number of nerve roots in the sections. Slides were read by an investigator (MP) who was unaware of the immunization or treatment protocol pertaining to the specimens.

The proximal portion of the cauda equine (± 2 cm) was snap-frozen in methylbutane cooled in liquid nitrogen and stored at -70°C. Eight micron cross-sections were cut on a cryostat, fixed in ice cold ether and used for immunocytochemistry.

### EXAMPLE 23

### Immunocytochemistry

Immunocytochemistry was performed using the following antibodies: ED1 1:1000 (macrophages), W3/13 (lymphocytes; Harlan Bioproducts for Science, IN), CD11b/c 1:100 (CR3 on macrophages, granulocytes, dendritic cells; Pharmingen, CA), IFN-γ 1:10 (Genzyme, MA). Eight micron frozen sections were blocked in normal rabbit serum for 30 minutes, then incubated with the primary antibody for 1 hour, washed with PBS, incubated with biotinylated secondary antibody for 1 hour, washed in PBS, incubated with ABC peroxidase (Vectastain) for 45 minutes, washed and developed with diaminobenzidine and hydrogenperoxide. Blocking with 0.01% hydrogenperoxide preceded incubation with primary antibodies to IFN-γ. The intensity and distribution of staining in nerve roots was scored as 1+, mild; 2+, moderate and 3+, severe.

### EXAMPLE 24

### Clinical symptoms

Rats started losing weight at day 10 after immunization and the first neurological symptoms appeared on day 12 while recovery started at day 16 or later. Eleven of 22 rats (50%) developed severe experimental allergic neuritis with a clinical score of 4 (severe paraparesis) or more and 2 out of 11 mock-IFN fed animals died on day 14 and 15 after immunization, while none of the IFN-α/β fed animals died. The clinical score in the mock-IFN fed rats (n=11, score 4.1 ± 2.4; experimental allergic neuritis ± SD) was significantly higher than in the IFN-α/β fed rats (n=11, score 3.3 ± 1.4; p<0.03, Wilcoxon rank test). More rats in the mock-IFN fed group developed a clinical score of 4 or greater (Figure 9A). Similarly more mock-IFN fed animals lost more than 25% of their body weight than IFN-α/β fed animals (Figure 9B).

### EXAMPLE 25

### Cell proliferation and cytokines

Spleen cell proliferation to Con A from IFN-α/β fed animals was decreased compared to mock-IFN fed animals. (spleen: mock-IFN 118,114 ± 5,194 vs rat IFN-α/β 835 ± 94, mean ± SEM, p<0.006, t-test).

IFN-γ production after stimulation with SP26 by lymph node and spleen cells from mock-IFN fed rats was much higher than IFN-γ production by lymph node and spleen cells from IFN-α/β fed rats as measured by ELISA but the difference did not reach statistical significance (lymph node: 18.9 ± 2.4 ng/ml vs 11.9 ± 3.8 ng/ml; spleen: 11.9 ± 0.4 ng/ml vs 6.3 ± 1.8 ng/ml, mean ± SEM, mock-IFN fed vs rat IFN-α/β fed, p<0.08). No difference in IFN-γ production was observed after stimulation with Con A or *M.tuberculosis*.

### EXAMPLE 26

### Histology

Histology of Epon-embedded sections showed demyelination to be significantly less severe in rat IFN-α/β fed rats (score 0.88 ± 0.11, mean ± SD) compared to mock-IFN fed rats (score 1.1 ± 0.26; p<0.05, t-test) but inflammation was not significantly different in both groups of animals (score 1.0 ± 0.2 vs 1.18 ± 0.24).

Immunocytochemistry with CD11bc antibody showed large numbers of inflammatory cells both perivascular and diffusely infiltrating nerve roots of the cauda equine in both rat IFN-α/β fed and mock-IFN fed rats when sacrificed at day 20. The majority of these cells were phagocytic ED1 positive macrophages and no significant differences were observed between rat IFN-α/β fed rats and mock-IFN fed animals in the extent of inflammation.

A group of rats treated with either rat IFN-α/β (n=3) or mock-IFN (n=3) were sacrificed early in the course of the disease at day 13 after immunization. Immunocytochemistry was performed to evaluate IFN-γ production in situ, which is only seen up until day 14 in experimental allergic neuritis, as well as inflammation. Infiltration by ED1 positive macrophages and W3/13 positive lymphocytes was much less severe in IFN-α/β fed rats than in mock IFN fed controls. IFN-γ staining was present in areas of dense inflammation on the surface of inflammatory cells. Figure 10 shows that IFN-γ staining was markedly reduced in IFN-α/β fed rats concomitantly with less extensive inflammation.

Administration of oral species-specific IFN-α/β compared to mock-IFN reduced the severity of experimental allergic neuritis, causing a significant decrease in clinical disease score and less weight loss. Histological data, collected at day 20 after immunization, when recovery had started, showed less demyelination in the IFN-α/β treated group without altering the extent of inflammation. At day 13 after immunization, in situ IFN-γ production was reduced together with inflammation as evaluated by immunocytochemistry. Proliferation studies of draining lymph node and spleen cells showed reduced proliferation to Con A in IFN-α/β fed experimental allergic neuritis rats compared to mock-IFN fed controls. Cytokine analysis revealed reduced IFN-γ production after stimulation with SP26, a peripheral nerve myelin antigen capable of inducing experimental allergic neuritis. In combination, the present invention demonstrates that oral administration of IFN-α/β reduces the severity of experimental allergic neuritis by a reduction in IFN-γ production. Thus, the present invention shows an immunomodulatory effect of oral IFN-α/β on the host immune system.

The mechanism by which oral IFN-α/β acts on the immune system is unknown but may include effects on Peyer's patches in the gut-associated lymphoid tissue (GALT) where regulatory cells can be generated. IFN-γ may induce immunoregulatory factors derived from CD8+ T cells that are responsible for disease modification. The reduced IFN-γ production associated with oral IFN-α/β administration suggests a possible functional inhibition of systemic Th1-like T helper cells found in EAE that produce IFN-γ. This results in a diminution of T cell encephalitogenicity of actively or passively induced disease.

Parenteral IFN-γ administration has been shown to augment both myelin-induced and T-cell mediated experimental allergic neuritis with increased clinical signs and histological abnormalities and enhanced oxidative burst by macrophages, while the opposite effect was obtained by parenteral administration of anti-IFN-γ antibody. IFN-γ also induces MHC class I and II expression on Schwann cells *in vitro.* IFN-γ, an inflammatory cytokine released by Th1 CD4+ T-cells, can upregulate MHC Class II expression on macrophages and endothelial cells and activate macrophages that play an important role in myelin phagocytosis. Reduced IFN-γ expression and reduced inflammation was seen at the onset of clinical disease in IFN-α/β fed animals. Histological evaluation at the end of the disease process, when recovery had started, showed reduced demyelination but not inflammation in IFN-α/β fed animals. Decreased IFN-γ and inflammation in early stages and diminished demyelination at later stages of disease suggest a critical role for IFN-γ in the pathogenesis of experimental allergic neuritis. The inability to decrease inflammation in later stages of disease may be due to partial suppression of IFN-γ production, the influence of other cytokines and inflammatory mediators and the induction of rather severe experimental allergic neuritis.

In summary, oral interferon is a biological response modifier effective in modifying disease in another experimental autoimmune disease, experimental allergic neuritis. The results of the present invention in experimental allergic neuritis showing reduced disease in rats fed IFN-α/β illustrate that oral IFN-α/β administration would be useful in the treatment of human immune-mediated neuropathies as Guillain-Barré syndrome or chronic inflammatory demyelinating polyradiculoneuropathy (CIDP).

All patents and publications mentioned in this specification are indicative of the levels of those skilled in the art to which the invention pertains. Any patents and publications are herein incorporated by reference to the same extent as if each individual publication was specifically and individually indicated to be incorporated by reference.

One skilled in the art will readily appreciate that the present invention is well adapted to carry out the objects and obtain the ends and advantages mentioned.

## Claims

1. The use of a type one interferon in the manufacture of a medicament for use in the treatment of an auto-immune disease by oral administration, **characterised in that** said interferon is in a dosage of from 50 IU/kg to 25,000 IU/kg and **in that** said interferon is ingested after administration.

2. Use as claimed in claim 1, wherein said interferon is selected from alpha-interferon and beta-interferon.

3. Use as claimed in claim 1 or claim 2, wherein said interferon is selected from human recombinant interferon, rat interferon and murine interferon.

4. Use as claimed in any one of claims 1 to 3, wherein said auto-immune disease is selected from multiple sclerosis, rheumatoid arthritis, diabetes mellitus, psoriasis, organ-specific autoimmune diseases, chronic inflammatory demyelinating polyradiculoneuropathy and Guillain-Barré syndrome.

5. The use of a type one interferon in the manufacture of a medicament for use in decreasing the severity or frequency of a relapse of multiple - sclerosis in a human by oral administration, **characterised in that** said interferon is ingested after administration.

6. Use as claimed in claim 5, wherein said interferon is selected from alpha-interferon and beta-interferon.

7. Use as claimed in claim 5 or claim 6, wherein said interferon is selected from human recombinant interferon, rat interferon and murine interferon.

8. The use of a type one interferon in the manufacture of a medicament for use in reducing inflammation associated with an auto-immune disease by oral administration, **characterised in that** said interferon is in a dosage of from 50 IU/kg to 25,000 IU/kg and **in that** said interferon is ingested after administration.

9. Use as claimed in claim 8, wherein said interferon is selected from alpha-interferon and beta-interferon.

10. Use as claimed in claim 8 or claim 9, wherein said interferon is selected from human recombinant interferon, rat interferon and murine interferon.

11. Use as claimed in any one of claims 8 to 10, wherein said auto-immune disease is selected from multiple sclerosis, rheumatoid arthritis, diabetes mellitus, psoriasis, organ-specific autoimmune diseases, chronic inflammatory demyelinating polyradiculoneuropathy and Guillain-Barré syndrome.

12. The use of a type one interferon in the manufacture of a medicament for use in inhibiting the onset of an auto-immune disease by oral administration, **characterised in that** said interferon is ingested after administration and **in that** said interferon is in a dosage of from 50 IU/kg to 25,000 IU/kg.

## Patentansprüche

1. Verwendung eines Interferons Typ 1 bei der Herstellung eines Medikaments zur Verwendung bei der Behandlung einer Autoimmunkrankheit durch orale Verabreichung, **dadurch gekennzeichnet, dass** das Interferon in 50 IU/kg bis 25.000 IU/kg dosiert ist, und dass das Interferon nach der Verabreichung durch Ingestion aufgenommen wird.

2. Verwendung nach Anspruch 1, wobei das Interferon aus Alpha-Interferon und Beta-Interferon ausgewählt ist.

3. Verwendung nach Anspruch 1 oder 2, wobei das Interferon aus menschlichem rekombinantem Interferon, Ratteninterferon und Mäuseinterferon ausgewählt ist.

4. Verwendung nach einem der Ansprüche 1 bis 2, wobei die Autoimmunkrankheit aus multipler Sklerose, rheumatoider Arthritis, Diabetes mellitus, Psoriasis, organspezifischer Autoimmunkrankheiten, chronischer entzündlicher Demyelinisationspolyradikuloneurothapie und Guillain-Barré-Syndrom ausgewählt ist.

5. Verwendung eines Interferons Typ 1 bei der Herstellung eines Medikaments zur Verwendung bei der Senkung der Schwere oder Häufigkeit eines Rückfalls multipler Sklerose bei einem Menschen durch orale Verabreichung, **dadurch gekennzeichnet, dass** das Interferon nach der Verabreichung durch Ingestion aufgenommen wird.

6. Verwendung nach Anspruch 5, wobei das Interferon aus Alpha-Interferon und Beta-Interferon ausgewählt ist.

7. Verwendung nach Anspruch 5 oder 6, wobei das Interferon aus menschlichem rekombinantem Interferon, Ratteninterferon und Mäuseinterferon ausgewählt ist.

8. Verwendung eines Interferons Typ 1 bei der Herstellung eines Medikaments zur Verwendung bei der Reduzierung von mit einer Autoimmunkrankheit zusammenhängenden Entzündung durch orale Verabreichung, **dadurch gekennzeichnet, dass** das Interferon in 50 IU/kg bis 25.000 IU/kg dosiert ist, und dass das Interferon nach der Verabreichung durch Ingestion aufgenommen wird.

9. Verwendung nach Anspruch 8, wobei das Interferon aus Alpha-Interferon und Beta-Interferon ausgewählt ist.

10. Verwendung nach Anspruch 8 oder 9, wobei das Interferon aus menschlichem rekombinantem Interferon, Ratteninterferon und Mäuseinterferon ausgewählt ist.

11. Verwendung nach einem der Ansprüche 8 bis 10, wobei die Autoimmunkrankheit aus multipler Sklerose, rheumatoider Arthritis, Diabetes mellitus, Psoriasis, organspezifischer Autoimmunkrankheiten, chronischer entzündlicher Demyelinisationspolyradikuloneurothapie und Guillain-Barré-Syndrom ausgewählt ist.

12. Verwendung eines Interferons Typ 1 bei der Herstellung eines Medikaments zur Verwendung bei der Hemmung des Ausbruchs einer Autoimmunkrankheit durch orale Verabreichung, **dadurch gekennzeichnet, dass** das Interferon nach der Verabreichung durch Ingestion aufgenommen wird, und dass das Interferon in 50 IU/kg bis 25.000 IU/kg dosiert ist.

## Revendications

1. Utilisation d'un interféron de type un pour la fabrication d'un médicament destiné à être utilisé pour le traitement d'une maladie auto-immune par administration orale, **caractérisée en ce que** le dit interféron est à une dose de 50 UI/kg à 25 000 UI/kg et **en ce que** ledit interféron est ingéré après administration.

2. Utilisation selon la revendication 1, dans laquelle ledit interféron est choisi parmi l'interféron alpha et l'interféron bêta.

3. Utilisation selon la revendication 1 ou la revendication 2, dans laquelle ledit interféron est choisi parmi l'interféron humain recombinant, l'interféron de rat et l'interféron murin.

4. Utilisation selon l'une quelconque des revendications 1 à 3, dans laquelle ladite maladie auto-immune est choisie parmi la sclérose en plaques, la polyarthrite rhumatoïde, le diabète sucré, le psoriasis, des maladies auto-immunes spécifiques d'organe, la polyradiculoneuropathie chronique inflammatoire démyélinisante et le syndrome de Guillain-Barré.

5. Utilisation d'un interféron de type un pour la fabrication d'un médicament destiné à être utilisé pour diminuer la gravité ou la fréquence d'une rechute de sclérose en plaques chez un être humain par administration orale, **caractérisée en ce que** ledit interféron est ingéré après administration.

6. Utilisation selon la revendication 5, dans laquelle ledit interféron est choisi parmi l'interféron alpha et l'interféron bêta.

7. Utilisation selon la revendication 5 ou la revendication 6, dans laquelle ledit interféron est choisi parmi l'interféron humain recombinant, l'interféron de rat et l'interféron murin.

8. Utilisation d'un interféron de type un pour la fabrication d'un médicament destiné à être utilisé pour réduire l'inflammation associée à une maladie auto-immune par administration orale, **caractérisée en ce que** ledit interféron est à une dose de 50 UI/kg à 25 000 UI/kg, et **en ce que** ledit interféron est ingéré après administration.

9. Utilisation selon la revendication 8, dans laquelle ledit interféron est choisi parmi l'interféron alpha et l'interféron bêta.

10. Utilisation selon la revendication 8 ou la revendication 9, dans laquelle ledit interféron est choisi parmi l'interféron humain recombinant, l'interféron de rat et l'interféron murin.

11. Utilisation selon l'une quelconque des revendications 8 à 10, dans laquelle ladite maladie auto-immune est choisie parmi la sclérose en plaques, la polyarthrite rhumatoïde, le diabète sucré, le psoriasis, des maladies auto-immunes spécifiques d'organe, la polyradiculoneuropathie chronique inflammatoire démyélinisante et le syndrome de Guillain-Barré.

12. Utilisation d'un interféron de type un pour la fabrication d'un médicament destiné à être utilisé pour inhiber le début d'une maladie auto-immune par administration orale, **caractérisée en ce que** ledit interféron est ingéré après administration et **en ce que** ledit interféron est à une dose de 50 UI/kg à 25 000 UI/kg.
